Europäisches Patentamt

European Patent Office

Office européen des brevets

(11) Publication number: 0 326 046
A2

# EUROPEAN PATENT APPLICATION

(21) Application number: 89101030.8

(22) Date of filing: 21.01.89

(51) Int. Cl.⁴: C12N 15/00 , C12N 1/20 , C07H 21/04 , C12P 21/02 , //(C12N1/20,C12R1:08)

The applicant has filed a statement in accordance with Rule 28 (4) EPC (issue of a sample only to an expert). Accession number(s) of the deposit(s): IFO 14698, FERM BP-1664,IFO 14699,FERM BP-1663,FERM BP-1087,IFO 14728,FERM BP-1771,IFO 14729 and FERM BP-1772.

A request for correction of description and claim 12 has been filed pursuant to Rule 88 EPC. A decision on the request will be taken during the proceedings before the Examining Division (Guidelines for Examination in the EPO, A-V, 2.2).

(30) Priority: 25.01.88 JP 14038/88
29.02.88 JP 46747/88

(43) Date of publication of application:
02.08.89 Bulletin 89/31

(84) Designated Contracting States:
AT BE CH DE ES FR GB GR IT LI LU NL SE

(71) Applicant: Takeda Chemical Industries, Ltd.
27, Doshomachi 2-chome Higashi-ku
Osaka-shi Osaka, 541(JP)

Applicant: Udaka, Shigezo
24-3, Uezonocho 1-chome Meitoh-ku
Nagoya Aichi 465(JP)

(72) Inventor: Kakinuma, Atsushi
C2-505, 3 Takenodai
Nagaokakyo, Kyoto 617(JP)
Inventor: Nakahama, Kazuo
15-59, Nishinokyo
Nagaokakyo, Kyoto 617(JP)
Inventor: Udaka, Shigezo
24-3, Uezonocho 1-chome, Meitoh-ku
Nagoya Aichi 465(JP)
Inventor: Yamagata, Hideo
1-305, 22 Sonoyamacho 2-chome,Chikusa-ku
Nagoya Aichi 464(JP)
Inventor: Tsukagoshi, Norihiro
510, Idakadai 2-chome, Meitoh-ku
Nagoya Aichi 465(JP)
Inventor: Takagi, Hiroaki
8-9, Sangen-cho
Choshi Chiba 288(JP)

(74) Representative: von Kreisler, Alek,
Dipl.-Chem. et al
Patentanwälte Von Kreisler-Selting-Werner
Deichmannhaus am Hauptbahnhof
D-5000 Köln 1(DE)

(54) Production of human epidermal growth factor.

(57) The present invention provides a recombinant DNA which comprises a human EGF-encoding DNA linked to the 3′-terminal of a promoter region-containing DNA, said promoter region being derived from *Bacillus brevis*, a transformant of *Bacillus brevis* having a recombinant DNA which comprises a human EGF-encoding DNA linked to the 3′-terminal of a promoter region-containing DNA, and a method for producing human EGF which comprises (a) cultivating said transformant in a culture medium, (b) producing and accumulating human EGF in a culture broth, and (c) recovering human EGF from the culture borth.

The method for producing human EGF according to the present invention permits mass production of human EGF at high efficiency while stably maintaining the human EGF-encoding DNA in the transformant.

## Production of Human Epidermal Growth Factor

BACKGROUND OF THE INVENTION

The present invention relates to a recombinant DNA technique for human EGF (human epidermal growth factor) production, and, specifically, to a DNA comprising the human EGF gene, a transformant of *Bacillus brevis* having said DNA gene, and a production method for human EGF using said transformant.

Human EGF comprises 53 amino acids; its amino acid sequence is already known [Gregory, H.; Nature, *257*, 325 (1975)]. This substance possesses gastric acid secretion suppressing and epidermal cell growth promoting activities; it is therefore expected to work well as a therapeutic drug for gastric ulcer, burns etc., and mass production of recombinant human EGF by gene manipulation technology is desired.

Most investigations of recombinant DNA have so far been conducted using *Escherichia coli*; a wide variety of foreign genes have already been expressed in *Escherichia coli* cells. In this method, however, the gene expression product is accumulated in cells; much time and labor is needed to extract the desired product from the cells and purify it from the extract, and it is also difficult to obtain the desired product in a perfect form.

On the other hand, bacteria of the genus *Bacillus* have long been industrially utilized as producers for various extracellular enzymes. It is considered possible to make *Bacillus* bacteria extracellularly secrete the desired protein as a gene product by cloning promoters and signal peptide-encoding regions of genes for these extracellular enzymes, ligating the structural gene for the desired protein downstream from them, and introducing this ligation product to *Bacillus* bacteria. It is conjectured that the proteins secreted by *Bacillus* bacteria are first synthesized as precursors having a signal peptide comprising about 20 to 30 amino acid residues in the amino terminal side, and the signal peptide portion is then cut off by signal peptidase to give mature protein. *Bacillus amyloliquefaciens* α-amylase gene [I. Palva et al.; Gene, *22*, 229 (1983)], *Bacillus licheniformis* penicillinase gene [S. Chang et al.; Molecular Cloning and Gene Regulation in Bacilli, Academic Press, p. 659 (1982)], and *Bacillus subtilis* α-amylase gene [H Yamazaki et al.; Journal of Bacteriology, *56*, 327 (1983)] have already been cloned; there are some reports on the production of foreign proteins by secretion using these signal peptides.

For producing foreign proteins by secretion as mentioned above, mainly *Bacillus subtilis* is used as host. This microorganism, however, also produces large quantities of extracellular protease, which decomposes the foreign proteins produced by secretion using recombinant DNA technology; the amount of their accumulation decreases noticeably.

Udaka et al. found that a large number of *Bacillus brevis* strains produce little protease. They prepared secretion vectors using promoters of genes for major extracellular proteins [described respectively as "outer wall protein and middle wall protein", kintaigai shuyo tanpakushitsu" (major extracellular proteins), and "saibo hyoso tanpakushitsu" (cell surface layer proteins) in H. Yamagata et al., Journal of Bacteriology, *169*, 1239 (1987); N. Tsukagoshi, Journal of the Agricultural Chemical Society of Japan *61*, 68 (1987); Gazette for Japanese unexamined patent publication No. 201583/1987] of *Bacillus brevis* 47 [FERM P-7224; cf. Gazettes for Japanese unexamined patent publication Nos. 58074/1985 and 201583/1987], a strain which produces little protease, and regions encoding signal peptides for protein (middle wall protein), a major extracellular protein as described above, and succeeded in producing α-amylase [Gazette for Japanese unexamined patent publication No. 201583/1987; H. Yamagata et al.; Journal of Bacteriology, *169*, 1239 (1987)] and swine pepsinogen [S. Udaka, Proceedings of the 1987 meeting of the Agricultural Chemical Society of Japan, pp/ 837-838; N. Tsukagoshi, Journal of the Agricultural Chemical Society of Japan, *61*, 68 (1987)] by secretion using this strain.

Furthermore, Takagi et al. isolated *Bacillus brevis* HPD31 [NB: this strain is identical with the strain *Bacillus brevis* H102 (FERM BP-1087), described in the present specification], a Bacillus brevis strain which does not produce protease, and succeeded in producing heat-resistant amylase at high efficiency using the strain as host [Proceedings of the 1987 Meeting of the Agricultural Chemical Society of Japan, p. 27].

As for human EGF, it is reported to have been produced by secretion using as hosts *Escherichia coli* [H. Ohgai; Bio Industry, *3*, 875 (1986)] and *Saccharoimyces cerevisiae* [Anthony J. Brake et al.; Proceedings of the National Academy of Science, USA, *81*, 4642(1984)].

BRIEF EXPLANATION OF THE DRAWING

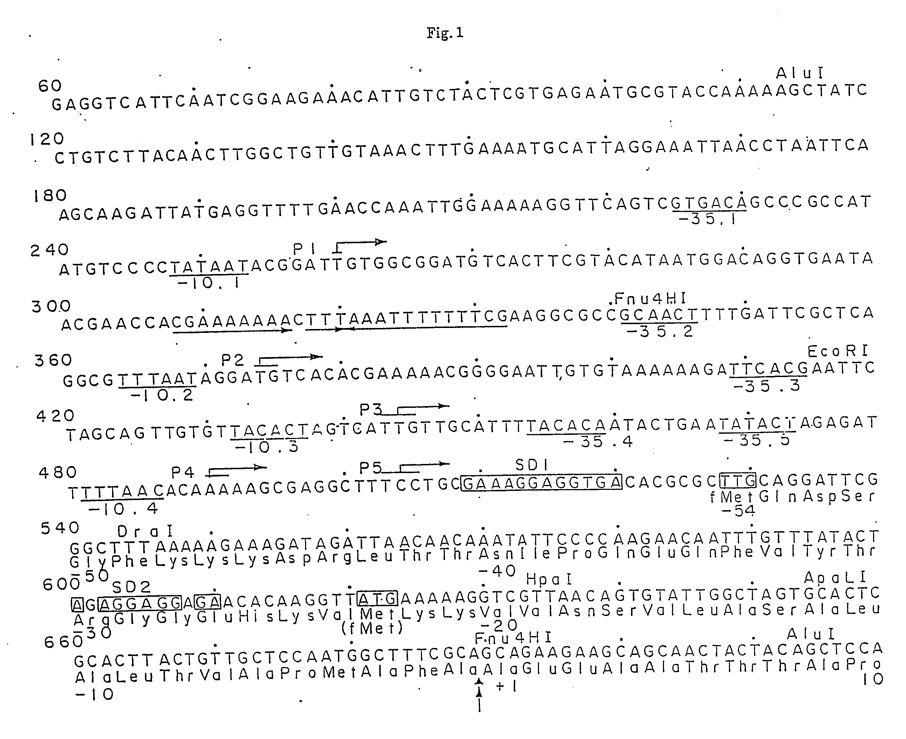

Fig. 1 shows the base sequence and amino acid sequence of the DNA containing the promoters of the genes for *Bacillus brevis* 47 major extracellular proteins and a region encoding the signal peptide for *Bacillus brevis* 47 MW protein and part of the N-terminal side of the mature protein, disclosed in Reference Example 1 (P represents a promoter; ↑ represents the signal peptidase cleavage site).

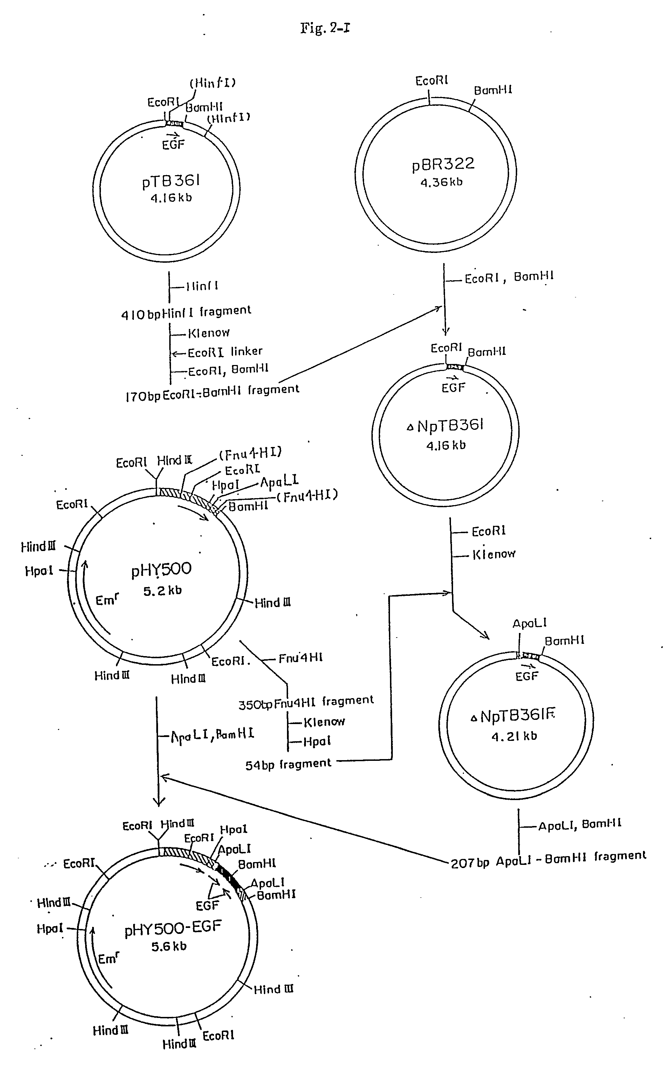

Fig. 2 shows a schematic chart of construction of the human EGF expression plasmids pHY500-EGF and pNU200-EGF31, disclosed in Examples 1 and 2 ( ▨▨▨▨ represents a DNA containing promoters and a signal peptide-encoding region; ▬▬▬. represents a human EGF-encoding DNA; Em[r] represents erythromycin resistance; ( ) represents a non-unique site).

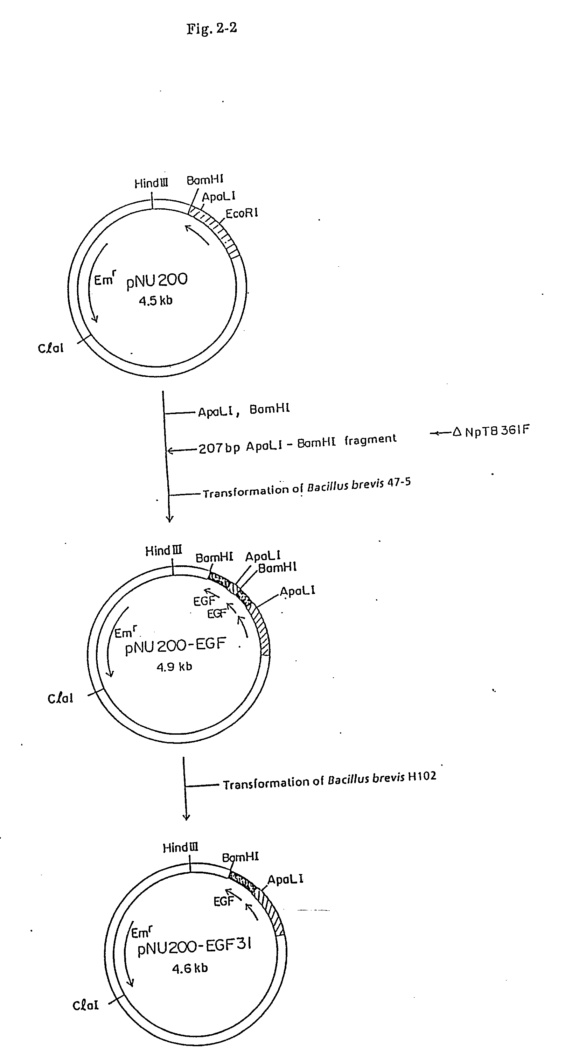

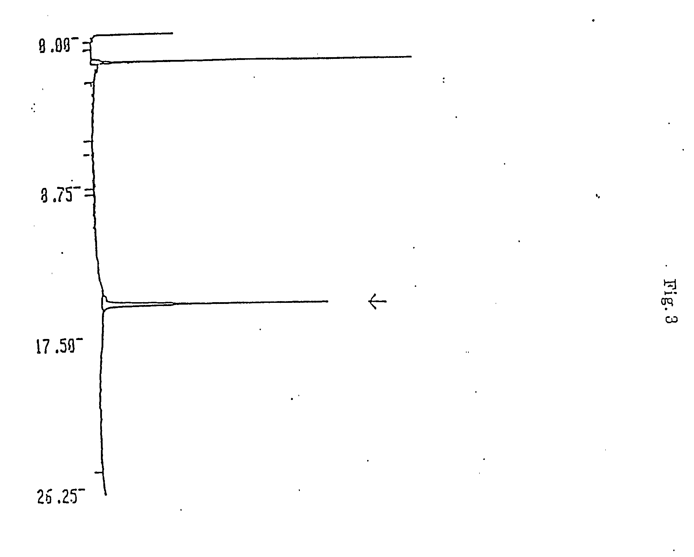

Fig. 3 shows the result of reversed phase high performance liquid chromatography obtained in Example 5 (the abscissa indicates retention time in min.; indicates the EGF elution time in min.).

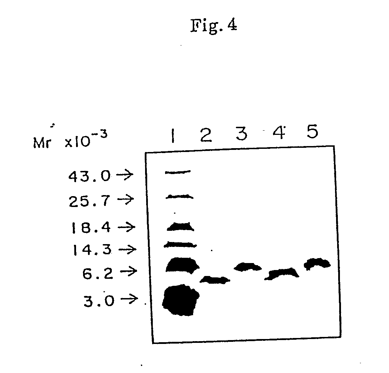

Fig. 4 shows the result of SDS-PAGE obtained in Example 5 (1 indicates the data on the molecular weight marker (under reducing conditions); 2 indicates the data on the standard preparation of purified human EGF (under reducing conditions); 3 indicates the data on the standard preparation of purified human EGF (under nonreducing conditions); 4 indicates the data on the authentic sample of human EGF (under reducing conditions); 5 indicates the data on the authentic sample of human EGF (under nonreducing conditions); the ordinate indicates molecular weight).

## DETAILED DESCRIPTION

As stated above, many attempts have separately been made to produce foreign proteins using *Bacillus brevis* as host or human EGF using *Escherichia coli* or *Saccharomyces cerevisiae* as host. Accordingly, the object of the present invention is to provide a new method of human EGF production using *Bacillus brevis* as host.

The present inventors made investigations with the aim of developing a method permitting efficient production of human EGF, and found that noticeable amounts of human EGF can be produced in the culture medium, while stably maintaining the human EGF gene, by expressing the human EGF gene using *Bacillus brevis* as host. The present inventors made further investigations based on this finding, developing the present invention.

Accordingly, the present invention comprises:

(1) a recombinant DNA which comprises a human EGF-encoding DNA linked to the 3′-terminal of a promoter region-containing DNA, said promoter region being derived from *Bacillus brevis*, (2) a transformant of *Bacillus brevis* having a recombinant DNA which comprises a human EGF-encoding DNA linked to the 3′-terminal of a promoter region-containing DNA, and (3) a method for producing human EGF which comprises (a) cultivating the transformant described in (2) above in a culture medium, (b) producing and accumulating human EGF in a culture broth, and (c) recovering human EGF from the culture broth.

Any DNA can be used for encoding human EGF, as long as it is capable of encoding human EGF; mention may be made of DNAs chemically synthesized by known methods based on the known amino acid sequence of human EGF. Examples of such DNAs include the DNA species which is represented by the formula:

AACAGTGATTCAGAATGTCCTCTCTCACACGATGGATACTG.
CCTCCATGACGGCGTGTGTATGTATATTGAAGCACTAGACA
AATACGCATGCAACTGTAGTTGGCTATATGGTGAACGATGC
CAGTACCGAGATCTGAAATGGTGGGAACTGCGA,

and which is synthesized in accordance with the methods described in the official gazettes for Japanese published unexamined patent publication Nos. 88881/1986 and 40290/1987 which correspond to EP 177915 and in Taniyama et al., Takeda-Kenkyusho-Ho, *45*, 136 (1986).

Any promoter can be used, as long as it functions in *Bacillus brevis* cells. *Bacillus brevis*-derived promoters are preferred; examples include promoters of *Bacillus brevis* 47 or *Bacillus brevis* H102 genes for their major extracellular proteins. Said promoters may be contained simply or in combination.

The recombinant DNA containing the promoter region must contain an SD sequence, a translation initiation codon etc. as well as the above-mentioned promoter(s),; it may further contain part of major extracellular protein genes.

For the present invention, human EGF may be accumulated in, or outside, *Bacillus brevis* cells. However, for facilitating human EGF extrac tion and purification and increasing productivities, it is desirable that the human EGF be extracellularly accumulated. In this case, the promoter region-containing DNA must also contain a signal peptide-encoding region in the 3′-terminal side.

Any signal peptide can be used, as long as it permits extracellular secretion of human EGF by *Bacillus*

3

*brevis*. Examples of such signal peptides include signal peptides for major extracellular proteins of *Bacillus brevis* 47 and *Bacillus brevis* H102; the signal peptide for *Bacillus brevis* 47 MW protein (middle wall protein) is particularly preferred.

Examples of the above mentioned promoter region and signal peptide-encoding region include 575 bp AluI-Fnu4HI fragment shown in Fig. 1, etc. and it may be chemically synthesized and also be prepared from *Bacillus brevis* 47-5 (pHY500-EGF) [IFO 14699, FERM BP-1663] or *Bacillus brevis* 475 (pNU200-EGF31) [IFO 14729, FERMBP-1772].

Any expression vector can be used for gene expression, as long as it functions in *Bacillus brevis* cells; examples include the plasmid pHY500, as obtained in Reference Example 1 presented below, and the plasmid pNU200, as obtained in Reference Example 2 presented below [S. Udaka; Journal of the Agricultural Chemical Society of Japan, *61*, 669 (1987)].

Any human EGF expression plasmid as prepared using the above-mentioned DNA can be used, as long as it functions in *Bacillus brevis* cells; examples include the plasmid pHY500-EGF, as obtained in Example 1 shown below, and the plasmid pNU200-EGF31, as obtained in Example 1 shown below.

For constructing these plasmids, there can be used, for example, the method described in "Molecular Cloning - A Laboratory Manual-", Cold Spring Harbor Laboratory (1982). Any microorganism can be used as host for constructing the plasmids, as long as it belongs to the species*Escherichia coli*, *Bacillus subtilis*, or *Bacillus brevis*; examples include *Escherichia coli* HB101, *Escherichia coli* DH1, *Bacillus subtilis* RM141 [Journal of Bacteriology, *158*, 1054 (1984)], *Bacillus brevis* 47 (FERM P-7224), and *Bacillus brevis* 47-5 (FERM BP-1664, IFO 14698).

Any *Bacillus brevis* can be used as host for hEGF gene expression; examples include *Bacillus brevis* 47 (FERM P-7224), *Bacillus brevis* 47-5 (FERM BP-1664, IFO 14698), and *Bacillus brevis* H102 (FERMBP-1087).

*Bacillus brevis* H102 (FERM BP-1087) is identical with *Bacillus brevis* HPD31 [Proceedings of the 1987 Meeting of the Agricultural Chemical Society of Japan, p. 27; S. Udaka, Journal of the Agricultural Chemical Society of Japan, *61*, 669 (1987)].

For transforming *Bacillus brevis*, known methods can be used; examples of usable methods include the method of Takahashi et al. [Journal of Bacteriology, *156*, 1130 (1983)] and the method of Chang and Cohen [Molecular and General Genetics, *168*, 111 (1979)].

Any medium can be used to cultivate the transformant thus obtained, as long as it permits the transformant to grow and produce the desired protein.

Carbon sources which can be used in the medium include glucose, sucrose, glycerol, starch, dextrin, molasses, urea, and organic acid. Nitrogen sources which can be used in the medium include organic nitrogen sources such as casein, polypeptone, meat extract, yeast extract casamino acid, amino acids (e.g. glycine) and NZ-amine; and inorganic nitrogen sources such as ammonium sulfate, ammonium chloride and ammonium nitrate. Inorganic salts such as potassium chloride, potassium hydrogenphosphate, potassium dihydrogenphosphate, sodium chloride and magnesium sulfate may also be added as necessary. Synthetic media composed mainly of sugar and inorganic nitrogen sources may also be used for the cultivation. When a strain exhibiting auxotrophy is used, the nutrients essential for its growth must be added to the medium. Examples of the nutrients include amino acids, vitamins and nucleic acid bases.

Antibiotics (e.g. penicillin, erythromycin, chloramphenicol, bacitracin, D-cycloserine) are added to the medium, if necessary for the cultivation. Defoaming agents (e.g. soybean oil, lard) may also be added as needed.

The initial pH of the medium is 5.0 to 9.0, preferably 6.5 to 7.5.

Cultivation temperature is normally 15 to 42° C, preferably 24 to 37° C. Cultivation time is normally 16 to 166 hours, preferably 24 to 96 hours.

After completion of the cultivation, cells and supernatant are separated by a known method, such as centrifugation or filtration. For extracting human EGF produced in the cells, the cells are ruptured by a standard method used in the present field, e.g. ultrasonic disruption, disruption using French press etc., mechanical disruption such as grinding, or disruption using cell wall lytic enzyme; a surfactant, such as Triton-X100 or deoxycholate, is added, if necessary. The human EGF-containing culture supernatant or extract thus obtained is subjected to ordinary processes of protein purification, e.g. salting-out, isoelectric precipitation, gel filtration, ion exchange chromatography, the hydroxyapatite method, high performance liquid chromatography (HPLC, FPLC etc.) to yield the desired human EGF.

The human EGF thus obtained can be quantified by radio-immunoassay using a kit commercially available from Amersham Co., UK, fibroblast receptor assay [Proceedings of the National Academy of Science, USA, 72, 1371 (1975)], or HPLC. It is also possible to quantify the human EGF on the basis of the BALB/C 3T3-A31 cell growth promoting activity [Journal of Cellular Physiology, *86*, 593 (1975)] or the HSC-

4

cell growth inhibitory activity [Journal of Cell Biology, *93*, 1 (1982)].

The production method for human EGF according to the present invention permits mass production of human EGF at high efficiency while stably maintaining the human EGF gene in the transformant; it is therefore useful in producing human EGF for use in therapeutic drugs, as well as clarifying the role of human EGF *in vivo*, the mechanism of cell growth regulation, and even the mechanism of carcinogenesis.

Furthermore, the method of the present invention also permits extracellular production of human EGF, thus affording easier collection of the desired human EGF.


EXAMPLES

The present invention will now be described in more detail by means of the following reference examples and working examples, but the present invention is not to be limited to these examples.

*Bacillus brevis* 47-5, disclosed in Reference Example 1, has been deposited at the Institute for Fermentation, Osaka (IFO), Japan under the accession number IFO 14698, since January 12, 1988; it has also been deposited at the Fermentation Research Institute (FRI), Agency of Industrial Science and Technology, Ministry of International Trade and Industry, Japan under the accession number FERM BP-1664, since January 20, 1988. Also, *Bacillus brevis* 47-5 (pHY500-EGF), disclosed in Example 1, has been deposited at the IFO under the accession number IFO 14699, since January 12, 1988, and at the FRI under the accession number FERM BP-1663, since January 20,1988.

*Bacillus brevis* H102 (a strain identical with *Bacillus brevis* HPD31, described in the Proceedings of the 1987 Meeting of the Agricultural Chemical Society of Japan, p. 27; S. Udaka, Journal of the Agricultural Chemical Society of Japan, *61*, 669 (1987)], disclosed in Example 2, has been deposited at the Fermentation Research Institute (FRI), Agency of Industrial Science and Technology, Ministry of International Trade and Industry, Japan under the accession number FERM BP-1087, since June 24, 1986. Also, *Bacillus brevis* 47-5 (pNU-200EGF), disclosed in Example 2, has been deposited at the Institute for Fermentation, Osaka (IFO), Japan under the accession number IFO 14728, since February 19, 1988; it has also been deposited at the FRI under the accession number FERM BP-1771, since February 29, 1988.

*Bacillus brevis* 47-5 (pNU200-EGF31), obtained by transforming *Bacillus brevis* 47-5 (FERM BP-1664, IFO 14698) by the method of Takahashi et al. [Journal of Bacteriology, *156*, 1130 (1983)] using the human EGF expression plasmid pNU200-EGF31, disclosed in Example 2, has been deposited at the IFO under the accession number IFO 14729, since February 19, 1988, and at the FRI under the accession number FERM BP-1772 since February 29,1988.

The abbreviations for amino acids etc., used in the present specification and drawings are based either on the abbreviations specified by the IUPACIUB Commission on Biochemical Nomenclature or those used commonly in related fields, as shown below. It should be noted that when there is a possibility of the presence of an optical isomer in amino acids, the isomer is an L-form unless otherwise stated.

Table 1

| DNA | : | Deoxyribonucleic acid |
|-----|---|------------------------|
| A | : | Adenine |
| T | : | Thymine |
| G | : | Guanine |
| C | : | Cytosine |
| SDS | : | Sodium dodecyl sulfate |
| Gly | : | Glycine |
| Ala | : | Alanine |
| Val | : | Valine |
| Leu | : | Leucine |
| Ile | : | Isoleucine |
| Ser | : | Serine |
| Thr | : | Threonine |
| Met | : | Methionine |
| Glu | : | Glutamic acid |
| Asp | : | Aspartic acid |
| Lys | : | Lysine |
| Arg | : | Arginine |
| His | : | Histidine |
| Phe | : | Phenylalanine |
| Tyr | : | Tyrosine |
| Trp | : | Tryptophan |
| Pro | : | Proline |
| Asn | : | Asparagine |
| Gln | : | Glutamine |

Reference Example 1

(Construction of the plasmid pHY500)

The plasmid pTT5 (6.1 kb) [Journal of Bacteriology, *158*, 1054 (1984)] was cleaved with the restriction enzyme Hind III at one site; this fragment was digested at both ends to an about one-sixth length with the nuclease Bal31, after which it was treated with the Klenow fragment (Large Fragment *E. coli* DNA Polymerase) to make both ends blunt and then treated with *Escherichia coli* alkaline phosphatase. Separately, a BamH I fragment containing a *Bacillus stearothermophilus*-derived fragment was isolated from the plasmid pHI301 (7.1 kb) [Tsukagoshi et al.; Molecular and General Genetics, *193*, 58 (1984)] and ligated with the above-mentioned pTT5-derived DNA fragment by means of T4 DNA ligase. The reaction mixture was used to transform *Bacillus subtilis* RM141 by the method of Chang and Cohen [Molecular and General Genetics, *168*, 111 (1979)]. From the kanamycin-resistant transformant thus obtained, a plasmid was isolated and named pTT-Am1 (7.2kb).

The plasmid pTT-Am1 thus obtained was treated with the restriction enzyme Hpa I for complete cleavage and with the restriction enzyme EcoR I for partial cleavage; this .fragment was treated with the Klenow fragment to make both ends blunt. Hind III linker was then added, followed by cyclization using T4 DNA ligase to yield the plasmid pTTAm2 (6.7 kb).

The plasmid pTT-Am2 was cleaved with the restriction enzymes Bgl II and Cla I; the smaller fragment was removed and the Bgl II-Cla I fragment of the plasmid pBAM101 (6.8 kb) [Tsukagoshi et al.; Journal of Bacteriology, *164*, 1182 (1985)] was inserted in place of the smaller fragment to yield the plasmid pTT-Am3 (7.4 kb).

The above plasmid pTT-Am3 was cleaved with the restriction enzyme Pvu II; this fragment was digested with the nuclease Bal31 and treated with the Klenow fragment to make both ends blunt, after which it was provided with Hind III linker. This DNA fragment was then cleaved with the restriction enzyme Hind III, after which it was cyclized using T4 DNA ligase to yield the plasmid pTT-Am300 (5.7 kb).

The plasmid pTT-Am300 was cleaved with EcoR I and cyclized using T4 DNA ligase to yield the kanamycin-resistant plasmid pFK1 (2.6 kb). The plasmid pHY481 (3.7 kb) [Yamagata et al., Applied and Environmental Microbiology, *49*, 1076 (1985); Gazette for Japanese unexamined patent publication No. 188073/1985 (cf. FERM P-7531)] was cleaved with the restriction enzyme Hpa II; the smaller fragment was removed and the larger Tag I fragment of the above-mentioned pFK1 was inserted in place of the above smaller fragment to yield the plasmid pFK2 (5.2 kb), which are resistant to both erythromycin and kanamycin.

The plasmid pFK2 was treated with hydroxylamine to induce mutation and used to transform *Bacillus brevis* 47-5 (FERM BP-1664, IFO 14698). From the transformants resistant to both erythromycin and kanamycin thus obtained, was selected the strain which showed an increased number of plasmid copies, from which a plasmid was isolated, and we named it pFK2copT (5.2 kb).

The Hpa II smaller fragment was removed from the above plasmid pFK2copT; the larger fragment was treated with the Klenow fragment to make both ends blunt, after which it was ligated with a mixture (1:1) of BamH I linker and Bgl II linker.

From the plasmid pCWP1 (7.5 kb) [Yamagata et al., Journal of Bacteriology, *169*, 1239 (1987); N. Tsukagoshi, Journal of the Agricultural Chemical Society of Japan, *61*, 68 (1987); Official Gazette for Japanese unexamined patent publication No. 201583/1987, which corresponds to GB 2182664], a 600 bp Alu I fragment (see Fig. 1) containing the promoters of the genes for *Bacillus brevis* 47 (FERM P-7224) major extracellular proteins and a region encoding the signal peptide for *Bacillus brevis* 47 MW protein and part of the N-terminal side of the mature protein, was isolated and ligated with the above-mentioned pFK2copT-derived DNA fragment by means of T4 DNA ligase. This reaction mixture was used to transform *Bacillus brevis* 47-5 by the method of Takahashi et al. [Journal Bacteriology, *156*, 1130 (1983)]. A plasmid was isolated from the transformant found resistant to erythromycin alone thus obtained, and which we named pHY500 (5.2 kb).

Reference Example 2

(Construction of the expression vector pNU200)

A 600 bp Alu I fragment containing the promoters of the genes for *Bacillus brevis* 47 major extracellular proteins and a region encoding the signal peptide for MW protein and part of the N-terminal side of the mature protein, was isolated from the plasmid pCWP1 (7.5 kb) [Yamagata et al., Journal of Bacteriology, *169*, 1239 (1987); N. Tsukagoshi, Journal of the Agricultural Chemical Society of Japan, *61*, 68 (1987); Official Gazette for Japanese unexamined patent publication No. 201583/1987, which corresponds to GB 2182664]. BamH I linker was then ligated to this fragment using T4 DNA ligase, followed by BamH I treatment to yield a 600 bp BamH I fragment.

The plasmid pHT-1 (5.6 kb) [Official Gazette for Japanese unexamined patent publication No. 58074/1985 (cf. FERM P-7226)] was cleaved with BamH I and Bgl II; the larger fragment was ligated with the above-mentioned 600 bp BamH I fragment by T4 DNA ligase. This reaction mixture was used to transform *Bacillus brevis* 47 (FERM P-7224). From the erythromycin-resistant transformant thus obtained, a plasmid was isolated, and which we named pNU100 (4.5 kb) [N. Tsukagoshi; Journal of the Agricultural Chemical Society of Japan, *61*, 68 (1987)]. The plasmid pNU100 was partially digested with the restriction enzyme EcoR I, after which it was treated with the Klenow fragment (Large Fragment *E. coli* DNA Polymerase) and cyclized using T4 DNA ligase to yield the secretion vector pNU200 (4.5 kb) [S. Udaka; Journal of the Agricultural Chemical Society of Japan, *61*, 669 (1987)], which lacks one portion at the ECoR I site in comparison with the plasmid pNU100.

Example 1

(Construction of human EGF expression plasmid pHY500-EGF (see Fig. 2) and preparation of transformant)

From expression vector pHY500 as obtained in Reference Example 1, a 350 bp Fnu4H I fragment (see Fig. 1), containing a region encoding the promoters for major extracellular protein genes and the signal peptide of MW protein, was isolated. This fragment was treated with Klenow fragment (Large Fragment *E. coli* DNA Polymerase) to blunt both ends, then cleaved with restriction enzyme Hpa I; a 54 bp DNA fragment encoding the signal peptide region between the 6th Asn and the C-terminal and its vicinity was isolated.

Plasmid pTB361 containing a DNA fragment encoding human EGF, was constructed in accordance with the method described in the Official Gazette for Japanese unexamined patent publication No. 40290/1987, which corresponds to EP 177915 [cf. Taniyama et al., Takeda-kenkyusho-ho, *45*, 136 (1986)]. A 410 bp Hinf I fragment encoding human EGF was isolated from plasmid pTB361. The isolated fragment was ileated with Klenow fragment (Large Fragment *E. coli* DNA Polymerase) to blunt both ends and was ligated with EcoR I linker (CGGAATTCCG) by means of T4 DNA ligase, after which it was treated with restriction enzymes EcoR I and BamH I to yield a 170 bp EcoR I-BamH I fragment. Separately, a 377 bp EcoR I-BamH I fragment was removed from plasmid pBR322, and the above-mentioned 170 bp EcoR I-BamH I fragment was inserted instead to yield plasmid ΔNpTB361.

Plasmid ΔNpTB361 was cleaved at the EcoR I sites, present in the region encoding the human EGF N-terminal and its vicinity, and was blunted using a Klenow fragment. The DNA fragment thus obtained was ligated at the 5'-terminal with a 54 bp DNA fragment encoding the signal peptide region between the 6th Asn and the C-terminal and its vicinity (mentioned above) to yield plasmid ΔNpTB361F, in which the signal peptide and the DNA encoding human EGF were appropriately linked. From this ΔNpTB361F plasmid, a 207 bp APaL I-BamH I fragment encoding the region between the approximate middle region of the signal peptide and the human EGF C-terminal was isolated.

Separately, expression vector pHY500 was cleaved with restriction enzymes APaL I and BamH I. The fragment thus obtained was ligated with the above-mentioned 207 bp APaL I-BamH I fragment by means of T4 DNA ligase. This reaction mixture was used to transform *Bacillus brevis* 47-5 (FERM, BP-1664, IFO 14698) by the method described by Takahashi et al. Journal of Bacteriology, *156*, 1130 (1983)]. A plasmid was isolated from the erythromycin-resistant transformant thus obtained, and was named pHY500-EGF. This plasmid, unexpected in the initial design, was found to have two 207 bp APaL I-BamH I fragments, in opposite directions, at the APaL I sites of the plasmid pHY500. (Fig. 2). Among the transformant strains thus obtained, a stable strain, *Bacillus brevis* 47-5 (pHY500-EGF) (FERM BP-1663, IFO 14699), was selected and used for cultivation in Example 3.

## Example 2

(Construction of the human EGF expression plasmid pNU200-EGF31 (see Fig. 2) and preparation of transformants)

A 350 bp Fnu4H I fragment (see Fig. 1) containing the promoters of the genes for major extracellular proteins and a region encoding the signal peptide for MW protein, was isolated from the plasmid pHY500 as obtained in Reference Example 1. This fragment was treated with the Klenow fragment (Large Fragment *E. coli* DNA Polymerase) to make both ends blunt, after which it was cleaved with the restriction enzyme Hpa I; a 54 bp DNA fragment encoding the region starting at the 6th Asn in the signal peptide and terminating near the C-terminal was then isolated.

The plasmid pTB361, containing a human EGF-encoding DNA fragment, was constructed by the method described in the Official Gazette for Japanese unexamined patent publication No. 40290/1987, which corresponds to EP 177915 [cf. Taniyama et al.; Takeda-Kenkyusho-Ho, *45*, 136 (1986)]; a 410 bp Hinf I fragment encoding human EGF was then isolated from the plasmid pTB361. The fragment thus obtained was treated with the Klenow fragment (Large Fragment *E. coli* DNA Polymerase) to make both ends blunt; EcoR I linker (CGGAATTCCG) was then ligated to the fragment using T4 DNA ligase, followed by treatment with the restriction enzymes EcoR I and BamH I to yield a 170 bp EcoR I-BamH I fragment. A 377 bp EcoR I-BamH I fragment was removed from the plasmid pBR322 and the above-mentioned 170 bp EcoR I-BamH I fragment was inserted to yield the plasmid ΔNpTB361.

The plasmid ΔNpTB361 was cleaved at the EcoR I site, present in the region encoding the portion near the human EGF N-terminal, and treated with the Klenow fragment to make both ends blunt. To the DNA fragment thus obtained, at the 5'-terminal, a 54 bp DNA fragment encoding the region starting at the 6th Asn in the signal peptide and terminating near the C-terminal (mentioned above) was ligated to yield the plasmid ΔNpTB361F, in which the signal peptide and the human EGF-encoding DNA are properly ligated together. From this plasmid, NpTB361F, a 207 bp APaL I-BamH I fragment encoding the region starting near the middle of the signal peptide and terminating the human EGF C-terminal (Fig. 2-1) was then isolated.

The expression vector pNU200 as obtained in Reference Example 2 was cleaved with APaL I and BamH I, followed by ligation with a 207 bp APaL I-BamH I fragment isolated from the plasmid ΔNpTB361F using T4 DNA ligase; this reaction mixture was used to transform *Bacillus brevis* 47-5 (FERM BP-1664, IFO 14698) by the method of Takahashi et al. [Journal of Bacteriology, *156*, 1130 (1983)]. A plasmid was then isolated from the erythromycin-resistant transformant thus obtained, and named pNU200-EGF; the transformant carrying the plasmid pNU200-EGF was named *Bacillus brevis* 47-5 (pNU200-EGF) (FERM BP-1711, IFO 14728). Unlike in the initial design, the plasmid pNU200-EGF was found to carry two inserted 207 bp APaL I-BamH I fragments in the same direction between the APaL I and BamH I sites of pNU200-EGF as well as the remaining 68 bp APaL I-BamH I fragment of pNU200EGF in the opposite direction (Fig. 2-2).

The human EGF expression plasmid pNU200-EGF obtained above was used to transform *Bacillus brevis* H102 [FERM BP-1087; a strain identical with *Bacillus brevis* HPD31, described in the Proceedings of the 1987 Meeting of the Agricultural Chemical Society of Japan, P. 27 and S. Udaka, Journal of the Agricultural Chemical Society of Japan, *61*, 669 (1987)]. A plasmid was then isolated from the transformant thus obtained; structural analysis thereof revealed that it lacks the DNA fragment which had been inserted to pNU200-EGF as an extra. This plasmid was named pNU200-EGF31 (Fig. 2-2); the transformant carrying the plasmid pNU200-EGF31 was named *Bacillus brevis*H102 (pNU200-EGF31).

The plasmid pNU200-EGF31 was isolated from *Bacillus brevis* 47-5 (pNU200-EGF31) (FERM BP-1772, IFO 14729), obtained by transforming *Bacillus brevis* 47-5 (FERM BP-1664, IFO 14698) by the method of Takahashi et al. [Journal of Bacteriology, *156*, 1130 (1983)] using the above-mentioned plasmid pNU200-EGF31, by the alkali extraction method described in "Molecular Cloning - A Laboratory Manual- Cold Spring Harbor Laboratory (1982) to yield *Bacillus brevis* H102 (pNU200-EGF31), identical with the above-mentioned transformant.

## Example 3

(Cultivation of transformants and human EGF production)

(i) The transformant *Bacillus brevis* 47-5 (pHY500-EGF) as obtained in Example 1 and *Bacillus brevis* 47-5 (pHY500) as control were each subjected to shaking culture at 30°C for 2 days using a medium (pH 7.0) composed of 1% glucose, 0.4% yeast extract, 2% Polypepton, 0.5% meat extract, 5mM $MgCl_2$, 100 μg/ml uracil, and 10 μg/ml erythromycin.

The culture broth obtained above was centrifuged; the human EGF concentration of the supernatant was determined by reversed phase high perfomance liquid chromatography (HPLC), on the basis of the HSC-1 cell growth inhibitory activity [Journal of Cell Biology, *93*, 1 (1982)], and on the basis of the BALB/C 3T3-A31 cell growth promoting activity [Journal of Cell Physiology, *86*, 593 (1975)]. The human EGF standard sample used for the determination was purchased from Wakunaga Pharmaceutical Co., Ltd., Japan. The results are shown in Table 2.

Table 2

| Human EGF Production by Secretion by Transformants | | | |
|---|---|---|---|
| Transformant | Human EGF (mg/L) | | |
| | HPLC | Growth Inhibition | Growth Promotion |
| Bacillus brevis 47-5 (pHY500) | 0 | <0.5 | <0.01 |
| Bacillus brevis 47-5 (pHY500-EGF) | 15 | 24 | 18 |

The culture supernatant obtained above was subjected to SDS polyacrylamide gel electrophoresis according to the method of Laemmli [Nature, 227, 680 (1970)], followed by subjecting to western blotting using anti-human EGF serum (Wakunaga Pharmaceutical Co., Ltd., Japan) according to the method of Burnette [Analytical Biochemistry, 112, 195 (1981)]. As the results, human EGF was detected in the culture supernatant and the molecular weight of human EGF in the culture supernatant agreed with that of the human EGF standard sample.

(ii) The transformant Bacillus brevis H102 (pNU200-EGF31) as obtained in Example 2 and Bacillus brevis H102 as control were each subjected to shaking culture at 33°C for 3 days using a medium (pH 7.2) composed of 3% proteose peptone, 0.2% yeast extract, 0.01% $CaCl_2 \bullet 2H_2O$, 0.01% $MgSO_4 \bullet 7H_2O$, 0.001% $FeSO_4 \bullet 7H_2O$, 0.001% $MnSO_4 \bullet 4H_2O$, 0.0001% $ZnSO_4 \bullet 7H_2O$, 5% glucose, 0.3% glycine, and 10 μg/ml erythromycin. Independently, Bacillus brevis H102 (pNU200-EGF31) was subjected to shaking culture at 33°C for 4 days using a medium of the same composition as above except that 0.03% Tween 40 was contained in place of the 0.3% glycine.

The culture broth obtained above was centrifuged; the human EGF concentration of the supernatant was determined by reversed phase high performance liquid chromatography (HPLC) and on the basis of the HSC-1 cell growth inhibitory activity [Journal of Cell Biology, 93, 1 (1982)]. The human EGF standard sample used for the determination was purchased from Wakunaga Pharmaceutical Co., Ltd., Japan. The results are shown in Table 3.

Table 3

| Human EGF Production by Secretion by Transformants | | | |
|---|---|---|---|
| Transformant | Cultivation days | Human EGF (mg/L) | |
| | | HPLC | Growth inhibition |
| Bacillus brevis H102 | 4 | 0 | <0.5 |
| Bacillus brevis H102 (pNU200-EGF31) | 3 | 130 | 88 |
| Bacillus brevis H102 (pNU200-EGF31) | 4 | 101 | 98 |

(iii) The transformant Bacillus brevis H102 (pNU200-EGF 31) as obtained in Example 2 was subjected to shaking culture at 30°C for 6 days using 5' YC medium (pH 7.2) contained 3% proteose peptone, 0.2% yeast extract, 3% glucose, 0.01% $MgSO_4.7H_2O$, 0.001% $FeSO_4.7H_2O$, 0.001% $MnSO_4.4H_2O$, 0.0001% $ZnSO_4.7H_2O$ and 0.3% glycine.

The culture broth obtained above was centrifuged and thus obtained culture supernatant was subjected to SDS polyacrylamide gel electrophoresis according to the method of Laemmli [Nature, 227, 680 (1970)], followed by subjecting to western blotting using anti-human EGF serum (Wakunaga Pharmaceutical Co., Ltd., Japan) according to the method of Burnette [Analytical Biochemistry, 112, 195 (1981)]. As the results, the human EGF concentration of the supernatant was 240 mg per liter culture.

## Example 4

(Purification of human EGF)

A 5-mℓ portion of the *Bacillus brevis* H102 (pNU200-EGF31) culture supernatant obtained in Example 3-(ii) was pretreated with a $C_{18}$ cartridge (SEP-PAK®, Waters Co.), after which it was subjected to purification processes in the order of reversed phase HPLC (Waters Co.), ion exchange HPLC (Toso Co., Japan), and reversed phase HPLC (Waters Co.) to yield 78 μg ofpurified human EGF.

## Example 5

(Properties of the purified human EGF)

The main properties of the purified human EGF obtained in Example 4 are as follows:

Reversed phase HPLC revealed that the obtained standard preparation of purified EGF showed one peak on HPLC (Fig. 3); its retention time corresponded to that of the commercially available authentic sample of human EGF (Wakunaga Pharmaceutical Co., Ltd., Japan).

Polyacrylamide gel electrophoresis using 18% polyacrylamide supplemented with 0.1% SDS (SDS-PAGE) revealed that the standard preparation exhibited the same behavior as that with the authentic sample, irrespective of whether the electrophoresis was conducted under reducing conditions or nonreducing conditions (Fig. 4).

An aliquot of the purified human EGF was placed in a glass test tube for hydrolysis and dried under reduced pressure; 5.7 N hydrochloric acid containing 4% thioglycolic acid was then added and the test tube was sealed under reduced pressure, followed by 24 hr hydrolysis at 110°C. After hydrolysis, the hydrochloric acid was evaporated under reduced pressure; the resulting residue was dissolved in 0.02 N hydrochloric acid and subjected to amino acid analysis. The results are shown in Table 4.

Table 4

| Amino Acid Composition | | | |
|---|---|---|---|
| Amino acid content | | Found value[1] | Calculated value |
| Asx[3] | 0.0828(μ mol) | 6.73 | 7 |
| Ser | 0.0319 | 2.59 | 3 |
| Glx[4] | 0.0617 | 5.02 | 5 |
| Pro | 0.0095 | 0.77 | 1 |
| Gly | 0.0509 | 4.14 | 4 |
| Ala | 0.0272 | 2.21 | 2 |
| Cys | -[2] | -[2] | 6 |
| Val | 0.0358 | 2.91 | 3 |
| Met | 0.0123 | 1.00 | 1 |
| Ile | 0.0263 | 2.14 | 2 |
| Leu | 0.0620 | 5.04 | 5 |
| Tyr | 0.0580 | 4.72 | 5 |
| Lys | 0.0250 | 2.03 | 2 |
| His | 0.0238 | 1.93 | 2 |
| Arg | 0.0330 | 2.68 | 3 |
| Trp | 0.0298 | 2.42 | 2 |
| Note: | | | |

1) Calculated on the basis of the Met content, taken as 1.
2) Cys was not detected.
3) Asx represents both Asp and Asn.
4) Glx represents both Gln and Glu.

N-terminal amino acid sequencing by pulse liquid automatic Edman decomposition (Applied Biosystems Co., 477A model) revealed that the N-terminal amino acid sequence of the purified human EGF agreed with reference data [Gregory, H.; Nature, *257*, 325 (1975)] (Table 5).

Table 5

| N-terminal Amino Acid Sequence | | | | | | | | | |
|---|---|---|---|---|---|---|---|---|---|
| | 1 | 2 | 3 | 4 | 5 | 6 | 7 | 8 | 9 |
| Standard preparation | Asn | Ser | Asp | Ser | Glu | X | Pro | Leu | Ser |
| Reference data | Asn | Ser | Asp | Ser | Glu | Cys | Pro | Leu | Ser |
| X: Not identified. | | | | | | | | | |

Hydrazinolysis [Narita et al.; Journal of Biochemistry, *59*, 170 (1966)] revealed that the C-terminal amino acid of the purified human EGF was arginine (identified as ornithine), in agreement with reference data [Gregory, H.; Nature, *257*, 325 (1975)].

HSC-1 cell growth inhibition [Barnes, J.; Journal of Cell Biology, *93*, 1 (1982)] was assessed; the purified human EGF exhibited activities nearly equivalent to those of the authentic sample.

**Claims**

1. A recombinant DNA which comprises a human EGF-encoding DNA linked to the 3′-terminal of a promoter region-containing DNA, said promoter region being derived from *Bacillus brevis*.

2. The recombinant DNA according to claim 1, wherein the promoter region-containing DNA is derived from *Bacillus brevis*.

3 The recombinant DNA according to claim 1, wherein the promoter region is derived from *Bacillus brevis* 47, *Bacillus brevis* 47-5 or *Bacillus brevis* H102.

4. The recombinant DNA according to claim 1, wherein the promoter region is derived from *Bacillus brevis* 47.

5. The recombinant DNA according to claim 1, wherein the promoter region is a promoter region of major extracellular protein genes of *Bacillus brevis* 47.

6. The recombinant DNA according to claim 1, wherein the promoter region-containing DNA contains a signal peptide-encoding region in the 3′-terminal side.

7. The recombinant DNA according to claim 6, wherein the signal peptide-encoding region is derived from *Bacillus brevis* 47.

8. The recombinant DNA according to claim 6, wherein the signal peptide-encoding region is a signal peptide-encoding region for middle wall protein of *Bacillus brevis* 47.

9. The recombinant DNA according to claim 6, wherein the signal peptide comprises a peptide having the following amino acid sequence:

Met-Lys-Lys-Val-Val-Asn-Ser-Val-Leu-Ala-Ser-Ala-Leu-Ala-Leu-Thr-Val-Ala-Pro-Met-Ala-Phe-Ala.

10. The recombinant DNA according to claim 1, wherein the promoter region-containing DNA is 575 bp.AluI-Fnu4HI fragment shown in Fig. 1.

11. The recombinant DNA according to claim 1, wherein the human EGF-encoding DNA is a chemicaly synthesized DNA.

12. The recombinant DNA according to claim 1, wherein the human EGF-encoding DNA has the following DNA sequence:

AACAGTGATTCAGAATGTCCTCTCTCACACGATGGATACTG

·CCTCCATGACGGCGTGTGTATGTATATTGAAGCACTAGACA

AATACGCATGCAACTGTAGTTGGCTATATGGTGAACGATGC

CAGTACCGAGATCTGAAATGGTGGGAACTGCGA,

13. The recombinant DNA according to claim 1, which is plasmid pHY500-EGF.

14. The recombinant DNA according to claim 1, which is plasmid pNU200-EGF31.

15. A transformant of *Bacillus brevis* having a recombinant DNA which comprises a human EGF-encoding DNA linked to the 3′-terminal of a promoter region-containing DNA.

16. The transformant according to claim 15, wherein the promoter region is derived from *Bacillus brevis*.

17. The transformant according to claim 15, wherein the promoter region-containing DNA contains a signal peptide-encoding region in the 3′-terminal side.

18. The transformant according to claim 15, wherein *Bacillus brevis* is *Bacillus brevis* 47, *Bacillus brevis* 47-5 or *Bacillus Brevis* H102.

19. The transformant according to claim 15, which is *Bacillus brevis* 47-5 (pHY500-EGF).

20. The transformant according to claim 15, which is *Bacillus brevis* H102 (pNU200-EGF31).

21. A method for producing human EGF, which comprises (a) cultivating the transformant according to claim 15 in a culture medium,(b) producing and accumulating human EGF in a culture broth, and (c) recovering human EGF from the cultuer broth.

22. The method according to claim 21, wherein human EGF is accumulated in a culture medium.

23. The method according to claim 21, wherein the transformant is *Bacillus brevis* 47-5 (pHY500-EGF) or *Bacillus brevis* H102 (pNU200-EGF31)

Fig.1

AluI
60
GAGGTCATTCAATCGGAAGAAACATTGTCTACTCGTGAGAATGCGTACCAAAAAGCTATC

120
CTGTCTTACAACTTGGCTGTTGTAAACTTTGAAAATGCATTAGGAAATTAACCTAATTCA

180
AGCAAGATTATGAGGTTTTGAACCAAATTGGAAAAAGGTTCAGTCGTGACAGCCCGCCAT
                                                    -35.1

240
ATGTCCCCTATAATACGGATTGTGGCGGATGTCACTTCGTACATAATGGACAGGTGAATA
          -10.1

P1 →

300                                              .Fnu4HI
ACGAACCACGAAAAAAACTTTAAATTTTTTTCGAAGGCGCCGCAACTTTTGATTCGCTCA
              ──────────→    ──→                -35.2

360                                                    EcoRI
GGCGTTTAATAGGATGTCACACGAAAAACGGGGAATTGTGTAAAAAAGATTCACGAATTC
    -10.2                                          -35.3

P2 →

420                           P3 →
TAGCAGTTGTGTTACACTAGTCATTGTTGCATTTTACACAATACTGAATATACTAGAGAT
              -10.3                    -35.4        -35.5

480         P4 →        P5 →        SD1
TTTTAACACAAAAAGCGAGGCTTTCCTGCGAAAGGAGGTGACACGCGCTTGCAGGATTCG
-10.4                                                  fMetGlnAspSer
                                                       -54

540    DraI
GGCTTTAAAAAGAAAGATAGATTAACAACAAATATTCCCCAAGAACAATTTGTTTATACT
GlyPheLysLysLysAspArgLeuThrThrAsnIleProGlnGluGlnPheValTyrThr
                                        -40   HpaI          ApaLI
600-50 SD2
AGAGGAGGAGAACACAAGGTTATGAAAAAGGTCGTTAACAGTGTATTGGCTAGTGCACTC
ArgGlyGlyGluHisLysValMetLysLysValValAsnSerValLeuAlaSerAlaLeu
-30          (fMet)           -20
660                           Fnu4HI                    AluI
GCACTTACTGTTGCTCCAATGGCTTTCGCAGCAGAAGAAGCAGCAACTACTACAGCTCCA
AlaLeuThrValAlaProMetAlaPheAlaAlaGluGluAlaAlaThrThrThrAlaPro
                                        +1                    10
-10

Fig. 2-I

EP 0 326 046 A2

Fig. 2-2

Fig. 3

Fig. 4